# EUROPEAN PATENT APPLICATION

(11) **EP 3 021 244 A1**
(43) Date of publication of application: **18.05.2016**
(21) Application number: 14192833.3
(22) Date of filing: 12.11.2014
(51) Int. Cl.: G06F 19/00

(54) **Blood purification device feedback method**

(71) Applicant: B. Braun Avitum AG, 34212 Melsungen (DE)
(72) Inventor: Bocz, Máté, 1094 Budapest (HU); Béky, Zsófia, 1037 Budapest (HU)
(74) Representative: Winter, Brandl, Fürniss, Hübner, Röss, Kaiser, Polte - Partnerschaft mbB

(57) **Abstract**

A method for providing feedback on user actions in a blood purification device comprises the steps of loading a task list defining process actions to be carried out into a processing environment of the blood purification device, wherein the task list includes at least one task assigned to a predetermined device and/or user action, selecting a set of sensors and/or detectors from a plurality of sensors and/or detectors arranged in the blood purification device based on requirements of the task list, and for each task in the task list, querying the selected set of sensors and/or detectors, determining operational and/or device task states based on the sensor and/or detector outputs, and outputting a user feedback indication notifying the user on the determined task state. A blood purification device equipped with supporting hardware is arranged to carry out the method.

## Description

The invention relates generally to blood purification. More specifically, the invention relates to monitoring operations during blood purification and a generation of feedback as to whether or not an operation in a sequence of a plurality of operations is successfully completed.

Operating devices blood purification therapies, such as blood purification devices and the like, require several manual user actions, such as hanging solution bags or loading a kit on the front of the device. A general usability issue with these kind of medical devices is that the devices do not provide enough feedback to a user on whether the performed action has been successful or not.

In many cases the device can check the correctness and can warn the operator about a possible mistake. This mistake can arise from device failure or malfunction or use errors. In case the operator can correct them, the device can draw the operator's attention to the existence of the mistake and can imply that the operator needs to correct it.

For example, generally known systems and methods of using such systems include a dialysis system, a dialysis system controller operatively coupled to a filtration system, water purification system, dialysate preparation system and dialyzer system of the dialysis system, and a user interface communicatively coupled to the dialysis system controller. The user interface is configured to enable user interaction with the dialysis system and guide a user step-wise through set-up and shut-down of the dialysis system according to a predetermined protocol. The user interface communicates with the dialysis system controller to activate an alarm condition when a deviation from the pre-determined protocol is sensed.

Such systems and methods, however, only point a used toward a required feedback or action, which is then to be provided or set by the user itself, and thus only suggest the feedback. Accordingly, in the particular field of blood purification devices, such systems and methods are disadvantageous in that they lack giving feedback to an operator on the success of his actions and whether they are accepted by the device.

In view of the above, an object of the invention resides in providing a blood purification device having means providing feedback regarding the status of process steps during manipulation of the device and parts and/or components of the machine or device.

According to the invention, this object is accomplished by a method for providing feedback on user actions as defined in claim 1 and by a blood purification device as defined in claim 13. Advantageous further developments of the invention are subject of the accompanying dependent claims.

As operating blood purification devices requires several manual user actions as part of the use process, such as manipulating (e.g. loading, unloading, hanging and the like) disposables (e.g. kits, syringes, solution bags and the like), the basic idea underlying the invention resides in a device providing feedback (expressible as e.g. graphical icons) on the status of process steps when manipulating disposables and parts of the machine. Finally, a graphical user interface can provide clear indication regarding the correct completion of tasks and draws attention to the ones that require further user actions. Prior thereto, the machine detects the status of the operator activities and assigns different indicators to states such as, at least, in progress, i.e. when a detection is in progress and no result can be detected yet, completed, i.e. when the activity is detected to be successfully completed, failed, i.e. when the activity is detected to be completed but the result is not acceptable, and/or not started, i.e. when the detection has not been started.

Advantages of the invention result in at least that the operator is aware that the device can detect whether the required user actions have been successfully performed, the operator is aware whether the device is in progress of checking the correctness of the performed user action, the operator is aware whether the performed user action has been performed correctly, and the operator is aware whether further user action is required or the process step is complete.

Accordingly, the invention improves user recognition of a system status, provides help and hints to the user on user action completion, increases user confidence while using the device, and improves the quality of the human-computer interaction. Overall, it enhances the usability of the device.

Thus, according to an aspect of the invention, the object is accomplished by a method for providing feedback on user actions in a blood purification device, comprising the steps of loading a task list defining process actions to be carried out into a processing environment of the blood purification device, wherein the task list includes at least one task assigned to a predetermined device and/or user action; selecting a set of sensors and/or detectors from a plurality of sensors and/or detectors arranged in the blod purification device based on requirements of the task list; and for each task in the task list, querying the selected set of sensors and/or detectors; determining and operational and/or device task states based on the sensor and/or detector outputs; and outputting a user feedback indication notifying the user on the determined task state.

Preferably, the task list includes at least one task requiring manual user action and/or at least one task requiring internal and/or automatic device action. Tasks forming part of more complex processes and/or procedures may involve both user and machine action, e.g. in a case where the user initiates a task and the machine then completes the task. By including both such tasks in the task list, the user can also get feedback on operations carried out, or completed, by the machine.

Further preferably, different user feedback indications are, in the form of indicators, assigned to different task states, said indicators including at least an indicator for a not started state, an in progress state, a successfully completed state, and a failed state. Thereby, the user gets improved feedback on what is happening during the current process.

Further preferably, said user feedback indication is generated in a visual form, a tactile form and/or an audible form and output to a user feedback notification means, and wherein said visual form includes flashing, colored and/or size variable items output to a display means as the user feedback notification means, said tactile form includes vibration of at least a predetermined part, component or section of the blood purification device as the user feedback notification means, and said audible form includes speech and/or at least one distinguishable audio tone output to a speaker means as the user feedback notification means. In this manner, also impaired users can receive the intended feedback from the device. Also, an optional multiple output prevents a feedback from easily being overlooked and/or overheard, which is helpful in cases of critical alerts.

Still preferably, plural sets of sensors and/or detectors are predefined in accordance with a plurality of tasks included in the task list, and at least one predefined set of sensors and/or detectors is selected for at least one of the plurality of tasks. In a device having many sensors and/or detectors, not all of them are always required for performing a certain task. Selecting sensors and/or detectors task-specifically reduces wear and contributes to increased processing speed.

Also preferably, each task in the task list has its task specific set of sensors and/or detectors assigned, and only the assigned set of sensors and/or detectors is queried and/or processed in the operational and/or device task state determining step. In a device having many sensors and/or detectors, not all of them are always required for performing a certain task. Selecting sensors and/or detectors task-specifically reduces wear and contributes to increased processing speed.

Alternatively, a common set of sensors and/or detectors is assigned to all tasks in a task list, and only a task specific subset thereof is queried and/or processed for a given task in the operational and/or device task state determining step. In a device having many sensors and/or detectors, not all of them are always required for performing a certain task. Selecting a common set of sensors for all tasks can put and keep sensors requiring a certain ramp-up time or recovery time in a standby or ready state for general availability, and task-specific further operation then reduces wear and contributes to increased processing speed.

In addition preferably, a step of initially displaying the loaded task with all user feedback indications set to not started, if a detection process is not yet started, or in progress, if detection has started and a state determination result is not yet obtained, is provided. Such a step is advantageous in improving the awareness of the users as to whether there is happening anything at all.

Further preferably, plural tasks in the task list are processed in a predetermined order, and processing results including at least a determined state are stored into a memory for each task individually and/or all tasks in common. Advantageously, the predetermined order also provides sort of guidance to the user of what is next, so that the user can time-efficiently prepare for a following task while he is waiting for completion of a currently running task.

Advantageously, information on a processing and/or results thereof is collected during the operational and/or device task state determining step and made available to the user in the form of task specific detail feedback. If such additional information is presented using further process details, which may be accessible via an additional information symbol, the users' ability to understand why a shown state has been determined is improved, and wrong next and/or repeatedly wrong actions can be avoided.

In addition preferably, at least upon a determination of a failed state, an error processing, evaluation processing and/or testing processing is carried out, and the user is notified thereof by variably outputting the associated user feedback indication and/or by generating tactile feedback and/or audible feedback in a distinguishable manner. Thereby, the attention of a user toward failed states, which are likely to be more critical, is significantly increased.

Still preferably, a step of providing at least one intermediate task list entry point to a task determined as failed in the operational and/or device task state determining step. If a task is determined to have failed and if, therefore, the task must be performed again in order to successfully complete the overall process it belongs to, having direct access to the task is advantageous in that only theses task(s) can be repeated until they are successfully completed. In particular, if a user needs another person's help to complete an action, the other person can easily interact without having to reset the entire process and do it all over again. Also, a use for training purposes is conceivable.

Finally, according to another aspect of the invention, the object is also accomplished by a blood purification device comprising a plurality of sensors and/or detectors, processing means and a user feedback output means, wherein the blood purification device is arranged to carry out the method for providing feedback on user actions according to one of the preceding claims. Advantageously, the method can be operated on any blood purification device or machine equipped with supporting hardware and arranged to carry out the method.

Hereinafter, the invention will be described in more detail based on a preferred embodiment thereof and with reference to the attached drawings. In the drawings,
Fig. 1 schematically shows a flow chart of an operation of a blood purification device providing user feedback according to a preferred embodiment;
Fig. 2 schematically exemplifies graphical indications, or symbols, displayed as a result of sensor and/or detector value processing and being used as feedback to a user in the embodiment; and
Fig. 3A and 3B schematically illustrate a user display of an initial state of the blod purification device, where detection is started, and an intermediate state, where a status of process steps is indicated by the symbols shown in Fig. 2.

Generally referring to the preferred embodiment, the configuration thereof basically relies on supporting hardware provided in any blood purification device/machine or renal therapy related device benefiting from the embodied blood purification device feedback method. Such hardware can, therefore, in particular comprise sensors, detectors and other measurement means of which a state or status can be queried, polled and/or sampled, or that deliver a certain value during device operation, and on suitable means arranged to process such queried status and/or values and generate an output based on the processing result.

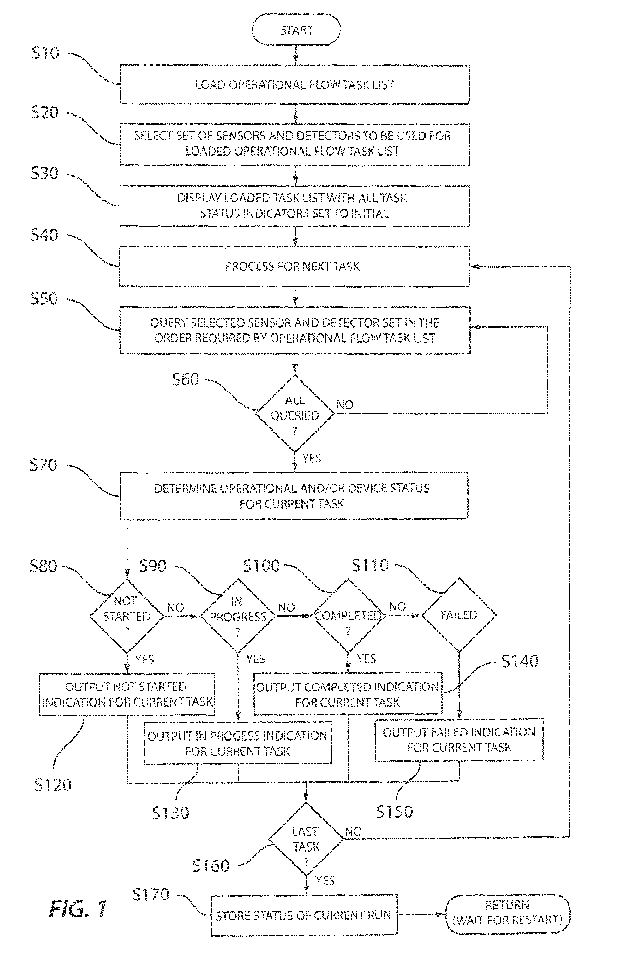

Referring to Fig. 1, which schematically shows a flow chart of an operation and method of a blood purification device providing user feedback according to a preferred embodiment, the subject method is in the following described.

In a first step S10, an operational flow task list is loaded into e.g. a process memory of a processing device of the underlying blood purification device or machine.

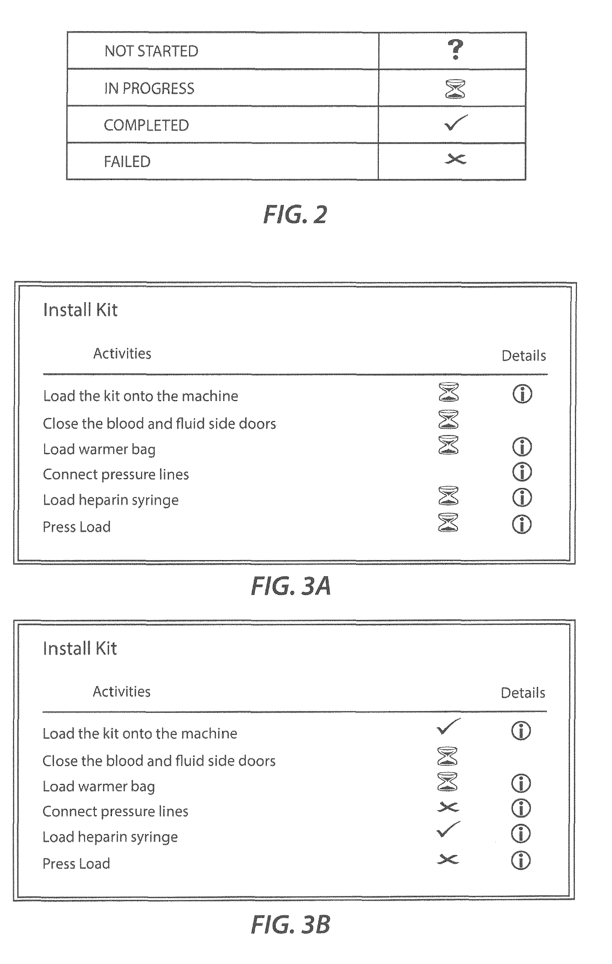

More specifically, if a user is to perform an action (or task) or a sequence of associated and/or subsequent actions (or tasks) at a blood purification device or machine during its operation, for which the device or machine is to generate user feedback, each action or sequence of actions has associated sort of a predetermined list of subsequent tasks to be carried out which is initialized by calling it from e.g. a memory, a table storage or the like. The list may be displayed in appropriate form so that the user can visually confirm the status of and feedback about his manipulation. An exemplary list of actions (having corresponding operational and/or processing steps underlaid) is shown in Fig. 3A and 3B, which will be described in more detail later on.

In a second step S20, a set out of sensors and/or detectors available in the device or machine and to be used for the action or task list loaded in step S10 is selected.

It is understood that a blood purification device or machine can be equipped with many sensors and detectors, of which however not all are always required to be used for a particular action. The sensor and/or detector selecting step S20 collects information on sensors and/or detectors required to be queried, polled and/or sampled for a selected action, i.e. sensors and/or detectors yielding measurements, signals, values and the like suitable to allow generation of the desired feedback to the user. It is understood that if such sensors are present in the device or machine, usually suitable and supportive processing means and components related thereto are also provided. A further explanation of such processing means and components is therefore omitted for reasons of convenience and simplification.

A third step S30 renders the loaded task list for display to the user. In this step, all displayed items may be classified as not started, incomplete or blanked out, until the user confirms the displayed step list as the one corresponding to his desired action or manipulation by e.g. pressing a confirmation button, or e.g. for a predetermined delay or waiting time until the device or machine starts working the task list.

In other words, the device or machine may be configured to start processing the operational flow only when the user confirms so. While this represents sort of a manual mode, alternatively a counter or the like may be provided rendering sort of a countdown giving the user a certain time to prepare for manipulation. The user is then aware that the machine will automatically start processing the task list after lapse of the countdown time.

Incomplete or blanked out in the above means that in the step 30 state of the operational flow, the display may indicate an "in progress" condition for all the individually displayed steps, or simply show a blank area which is beginning to fill and show state symbols only after processing has actually started. In the latter case, the user obtains additional feedback that the device or machine is still in a waiting, standby and/or energy saving state (i.e. temporarily halted at step S30).

In a step S40, the processing for the next task is started. The next task can be the first task (with no predecessor in the current processing), or a task subsequent to a previous one, for which the operational flow returns from a later routine section if not all tasks are processed and/or at least one task remains to be processed.

In a step S50, the set of sensors and/or detectors selected in step S20 is queried. There may be a certain predetermined order of query, if it is necessary to retrieve signals and/or values from one or more sensors in a particular order and/or at a specific time. In a simple case, all sensors and/or detectors may be queried as to their states, outputs and/or values at the same time when step S50 is carried out. It is understood that the results of step S50 may be stored into a memory or storage section for further processing, evaluation and/or protocol or history purposes.

In a step S60, it is verified whether or not all required sensors and/or detectors have been polled, sampled and/or queried. In the a.m. basic case, the result of step S60 is immediately "YES", while in other cases the flow may return to step S50 and process those sensors and/or detectors whose outputs are still required.

At step S60, an error processing, evaluation and/or testing processing (not shown) may additionally be involved in order to allow sensor and/or detector monitoring, verification and/or testing in order to ensure that the obtained outputs are valid or at least make sense for the intended purpose. In this way, defective or miscalibrated components and/or conditions that could falsify an obtained feedback result can be detected and replaced or compensated for.

In a step S70, after all sensors and/or detectors are queried, polled or sampled, an operational state or status is determined for the currently processed task of the task list.

In this step S70, the data obtained from the sensors and/or detectors are intelligently and/or mutually related to each other and classified as to whether an obtained final assertion indicates a predetermined status. For example, the obtained data can be logically combined, e.g. decided based on AND, OR logic and the like, derived from a series of if-then-conclusions, looked up in a table, and/or thresholded, just to name a few and without limitation thereto, and at the end converted into a unique statement on the status of the current step or task at that time.

In steps S80, S90, S100 and S110, a decision is made as to which indicator or symbol is to be output to the user as the feedback to be given.

A preferable sequence is e.g. to check in step S80 whether an action has been started at all. If, for example, during the installation of a kit to a device or machine, a next user action, or task/process step the user needs to complete, resides in "closing the blood and fluid side doors", and the process is waiting for any sensor and/or detector output indicating that the user has begun this task, the check in step S80 yields "YES" if there is no such indication and the process proceeds to a step S120 in which a symbol indicating "not started" is output to the user. If a corresponding indication that the user has begun to carry out his pending task is present, the check in step S80 yields "NO", and the process proceeds to step 90.

Then, in step S90, a check is made whether a task is in progress. Basically, any task requiring time, e.g. device processing time, set-up time, initialization time, or user time (for e.g. handling bags while replacing them) and having the machine and/or user wait for completion can result in "YES" in step S90 and an output of a corresponding "in progress" notification as the user feedback in a step S130. If the device or machine determines that the task is no longer pending, which may be the case if e.g. repeated sensor and/or detector polling yields stable data indicating no further change, a confirmation is detected by activation of e.g. an end switch, or a user presses a confirmation or "next" key and the like, step S90 yields "NO", and the process proceeds to step S100.

Next, in step S100, a completion verification process is carried out in which the device or machine determines whether or not the task, step or process has been completed successfully. If it is determined that the task, step or process has been successfully completed, the check in step S100 yields "YES" and an indication of "completed" is output in a step S140. If it is determined that the task, step or process has not been completed successfully, the check in step S100 yields "NO", and the process proceeds to step S100 setting a "failed" condition and state, and then to a step S150, in which the user is notified of the failure as the user feedback.

Either of the steps S120, S130, S140 and S150 is followed by a next step S160, in which it is checked whether or not the currently processed task was the last task in the currently worked operational flow task list. If "NO" in step S160, the process returns to step S40 and enters into the processing for the next task in the list. If "YES" in step S160, which indicates that all tasks in the currently loaded operational flow task list have been processed, the flow proceeds to a step S170, in which the status of the current process run is stored for further processing and/or use.

Thereafter, the flow can enter into a halt or waiting state until it is called again or restarted for another user action, manipulation or task. It is understood that a user action, manipulation or task may reside in an overall action, such as "Install Kit" as shown in Fig. 3A, and involve a number of "Activities" which may basically correspond to the operational flow task list in step 10 (where it is needless to say that in a practical embodiment such a list will of course comprise a more detailed step-and-substep-structure in underlying processing routines). In other words, steps S40 to S160 in Fig. 1 relate to the individual activities, which are processed one after another, with "Load the kit onto the machine" being a first operational flow step or task, and "Press Load" being a last operational flow step or task.

Specifically regarding the "failed" condition, the user may be presented an intermediate list entry and rerun opportunity via e.g. a user interface or a key, such as single step repetition until the failed state or condition is resolved. Alternatively, the entire operational flow may be worked again using the latest current run status stored in step S170. Using the latest current run status, tasks that were confirmed as successfully completed can be skipped and only tasks that had failed can be requested to be carried out again.

Referring to Fig. 3B, e.g. a pressure lines connection failure should not require to load the entire kit anew, so that the device or machine can determine from the stored current run status that the first task to be carried out in a flow rerun is the "connect pressure lines" task, and can skip the "load heparin syringe" task as well. In this way, failure conditions can be straightforwardly addressed, and failure elimination time can be shortened significantly.

Fig. 2 schematically exemplifies graphical indications, icons or symbols which are displayed as a result of sensor and/or detector value processing and as the feedback to a user.

More specifically, the "not started" symbol, or user feedback symbol, may be a question mark and displayed in response to a "YES" decision in step S80, the "in progress" symbol may be an hourglass and displayed in response to a "YES" decision in step S90, the "completed" symbol may be a positive check mark and displayed in response to a "YES" decision in step S100, and the failed symbol may be an X-symbol. The individual symbols may be presented in colors, with for example the check mark displayed in green and the X-symbol displayed in red, for simplified recognition by the user.

It is understood that other colors and/or symbols may be used as need be. It is further understood that the same feedback functionality can be provided by means different from graphical symbols. In other words, the device can, alternatively or additionally, and for all feedback items or for only part thereof, provide audible (e.g. beeps), tactile (e.g. vibrations) or other optical (e.g. changing the color of a light) feedback.

Fig. 3A and 3B schematically illustrate a user display of an initial state of the blood purification device, where detection is started, and an intermediate state, where a status of process steps is indicated by the symbols shown in Fig. 2.

In Fig. 3A and 3B, a process of a disposable kit installation is illustrated as an example and as it is presented to a user on e.g. a display or touch screen connected to the blood purification device or machine. The process consists of several tasks (i.e. process steps) the user needs to complete when installing the kit. More specifically, Fig. 3A depicts an initial state where detection is started but has not yet rendered any results, and Fig. 3B illustrates how the status of each process step is indicated by the symbols described with reference to Fig. 2 above.

In greater detail, the display or touch screen names the process in a first line on top of the screen, and shows an "Activities" column on the left and a symbol column and a "Details" column on the right of the screen. The "Details" column may be marked with sort of an information symbol indicating that further details or e.g. help regarding the associated process step may be retrieved. As can be seen from Fig. 3A, there may be process steps for which a symbol is lit already in the initial state, and process steps having no symbol but only a blank space instead. A reason for a display in this manner may reside in more complex tasks requiring the polling and processing of plural sensors and/or detectors and/or time, which are then marked "in progress", and tasks involving an immediate yes-or-no detection (such as e.g. whether pressure lines are connected or not), where there is no practical "in progress" state to be displayed.

The tasks forming part of the "Install Kit" process are listed in rows top-down on the screen, and preferably ordered in logical sequence just as they appear in practice. In other words, the user is guided from one task to a next one, and at the same time obtains quite immediate feedback on the state of the device or machine and the success or failure of its manipulation during which the individual symbols change accordingly, as shown in Fig. 3B, after leaving the initial state shown in Fig. 3A.

As described above, a general usability issue with known medical devices is that the operator does not get enough feedback on whether the performed action has been successful or not. Therefore the device described in here is arranged to provide information on the correctness of these actions if possible. Combining various sensor and detector values and their change over time, the device detects changes and intelligently determines what the operator has done. This translates to a successful or a failed user action. In the latter case, further actions are needed. The device also informs the operator whether it is currently checking the correctness or not. If yes, the operator anticipates that the device will provide a successful or failed result in a reasonably short time. If the device is currently not checking the activity then the operator knows that there is no need to wait for a result. The checking for correctness can be started manually or automatically by an algorithm. In order to provide understandable feedback to the user, generally understandable icons are used, e.g. green ticks, red crosses, and the like. The icons can also be animated (e.g. gif) to better express the active state of the device, i.e. to show that the device is working with this item.

While the invention has been described with reference to a preferred embodiment and the accompanying drawings, it is understood that the present invention is not in any way limited to particular details disclosed with respect to this preferred embodiment, and that any modification readily apparent to the skilled person based on the here presented teaching is deemed to be within the scope of protection as defined by the appended claims.

## Claims

1. A method for providing feedback on user actions in a blood purification device, comprising the steps of:
loading a task list defining process actions to be carried out into a processing environment of the blood purification device, wherein the task list includes at least one task assigned to a predetermined device and/or user action;
selecting a set of sensors and/or detectors from a plurality of sensors and/or detectors arranged in the blood purification device based on requirements of the task list; and
for each task in the task list,
querying the selected set of sensors and/or detectors;
determining operational and/or device task states based on the sensor and/or detector outputs; and
outputting a user feedback indication notifying the user on the determined task state.

2. A method according to claim 1, wherein the task list includes at least one task requiring manual user action and/or at least one task requiring internal and/or automatic device action.

3. A method according to claim 1 or 2, wherein different user feedback indications are, in the form of indicators, assigned to different task states, said indicators including at least an indicator for a not started state, an in progress state, a successfully completed state, and a failed state.

4. A method according to one of the preceding claims, wherein said user feedback indication is generated in a visual form, a tactile form and/or an audible form and output to a user feedback notification means, and wherein said visual form includes flashing, colored and/or size variable items output to a display means as the user feedback notification means, said tactile form includes vibration of at least a predetermined part, component or section of the blood purification device as the user feedback notification means, and said audible form includes speech and/or at least one distinguishable audio tone output to a speaker means as the user feedback notification means.

5. A method according to one of the preceding claims, wherein plural sets of sensors and/or detectors are predefined in accordance with a plurality of tasks included in the task list, and at least one predefined set of sensors and/or detectors is selected for at least one of the plurality of tasks.

6. A method according to one of the preceding claims, wherein each task in the task list has its task specific set of sensors and/or detectors assigned, and only the assigned set of sensors and/or detectors is queried and/or processed in the operational and/or device task state determining step.

7. A method according to one of the preceding claims, wherein a common set of sensors and/or detectors is assigned to all tasks in a task list, and only a task specific subset thereof is queried and/or processed for a given task in the operational and/or device task state determining step.

8. A method according to one of the preceding claims, further comprising a step of initially displaying the loaded task with all user feedback indications set to not started, if a detection process is not yet started, or in progress, if detection has started and a state determination result is not yet obtained.

9. A method according to one of the preceding claims, wherein plural tasks in the task list are processed in a predetermined order, and processing results including at least a determined state are stored into a memory for each task individually and/or all tasks in common.

10. A method according to one of the preceding claims, wherein information on a processing and/or results thereof is collected during the operational and/or device task state determining step and made available to the user in the form of task specific detail feedback.

11. A method according to one of the preceding claims, wherein at least upon a determination of a failed state, an error processing, evaluation processing and/or testing processing is carried out, and the user is notified thereof by variably outputting the associated user feedback indication and/or by generating tactile feedback and/or audible feedback in a distinguishable manner.

12. A method according to one of the preceding claims, further comprising a step of providing at least one intermediate task list entry point to a task determined as failed in the operational and/or device task state determining step.

13. A blood purification device comprising a plurality of sensors and/or detectors, processing means and a user feedback output means, wherein the blood purification device is adapted to carry out the method for providing feedback on user actions according to one of the preceding claims.
